# EUROPEAN PATENT APPLICATION

(11) **EP 1 878 411 A1**
(43) Date of publication of application: **16.01.2008**
(21) Application number: 05786112.2
(22) Date of filing: 28.07.2005
(51) Int. Cl.: A61F 9/00, G02C 7/04

(54) **THERAPEUTIC CONTACT LENS FOR PSEUDOAPHAKIC EYES AND/OR EYES UNDERGOING A NEURODEGENERATIVE PROCESS**

(30) Priority: 19.04.2005 ES 200500937
(71) Applicant: UNIVERSIDAD COMPLUTENSE DE MADRID, E-28040 Madrid (ES)
(72) Inventor: SÁNCHEZ RAMOS, Celia, Dpto. Óptica, 028040 Madrid (ES)
(74) Representative: Carpintero Lopez, Francisco
(86) International application number: PCT/ES2005/000431
(87) International publication number: WO 2006/111588

(57) **Abstract**

The invention relates to a contact lens for pseudo-aphakic eyes and/or eyes undergoing macular and retinal degeneration, **characterized by** resulting from applying a filter with yellow pigment to a standard contact lens for the purpose of protecting the eyes from the short wavelengths in the visible spectrum (less than 500 nm).

This invention eliminates the difficulties and risks of existing techniques to provide this protection to eyes subjected to cataract surgery and to improve protection in eyes undergoing neurodegenerative processes simply with the use of the contact lens.

The invention consists of the combination of a standard contact lens and yellow pigment filter which absorbs short wavelengths of 350-500 nm, both of which are suitable for use in the human eye.

## Description

### Object of the Invention

The invention relates to the ophthalmology field.

The object of the invention is a contact lens for pseudo-aphakic eyes (those subjected to cataract surgery) and/or undergoing macular and retinal degeneration, resulting from applying a filter with yellow pigment to a standard contact lens to protect the eyes from the short wavelengths in the visible spectrum (less than 500 nm).

### State of the Art

Visual perception is the result of the response to visible radiation, i.e. 380-760 nm. In the environment, solar radiation represents the main risk for vision. The sun emits UV rays and IR radiation which are mainly absorbed by the atmosphere. When solar radiation that is transmitted through the atmosphere reaches the surface of the Earth, it consists of UV-B rays (230-300 nm), UV or UV-A rays (300-380 nm), visible light (380-760 nm) and IR rays (760-1400 nm). In a normal state of health, the human eye transmits IR rays and most of the visible spectrum freely to the retina but the cornea and the crystalline lens prevent the most reactive wavelengths in the visible spectrum (UV-B rays and the portion of blue light in the visible spectrum) from reaching the retina.

For its part, the human crystalline lens changes its transmission characteristics with age, its yellow colour intensifying and its capacity to filter UV rays and blue light increasing. For this reason, in persons older than 65 years, violet light (< 400 nm) is not transmitted and the transmission of blue light (400-500 nm) notably decreases.

The retina is also able to protect itself from short wavelengths in two ways: through a heterogeneous distribution of photoreceptors, such that there are no photoreceptors sensitive to blue light in the macular depression; and through the action of yellow pigments existing in this area, which also exert a protective action.

This natural protection of the human eye against the shortest wavelengths of light, i.e. protection due to the crystalline lens and the retina, can be seriously affected by certain pathologies and/or surgical interventions:
- Cataracts, for which the only surgical treatment involves extracting the crystalline lens.
- A pathological aging process giving rise to retinal structure degradation, causing age-related macular degeneration (AMD), frequently occurs.

Moreover, persons over the age of 65 years with both pathologies, cataracts and AMD, must be kept in mind. Cataracts are the main cause of vision loss and AMD is the main cause of blindness in this population segment. In addition, due to the increase in life expectancy, the presumable increase in both pathologies must be considered, such that much interest has been generated in the field of research and its application in the optical industry.

Several epidemiological studies in the literature have evaluated the association between cataract surgery and age-related macular degeneration (AMD), including the works of Klein (Klein R, Klein BE, Wong TY, Tomany SC, Cruickshanks KJ. The association of cataract and cataract surgery with the long-term incident of age-related maculopathy. Arch Ophthalmol 120:1551-1558.2002) and Freeman (Freeman E, Muñoz B, West SK, Tielsch JM, Schein OD. Is there an association between cataract surgery and age-related macular degeneration. Am J Ophthalmol 135(6): 849-856.2003).

These authors argue that the risk of developing symptoms of AMD is greater in people subjected to cataract surgery. However, previous investigations by Wang (Wang JJ, Mitchell P, Cumming RG, Lim R. Cataract and age-related maculopathy: the Blue Mountains Eye Study. Ophthalmic Epidemiol 6: 317-326.1999) and McCarty (McCarty CA, Mukesh BN, Fu CL, Mitchell P, Wang JJ, Taylor HR. Risks factors for age-related maculopathy: The Visual Impairment Project. Arch Ophthalmology 119:1455-1462.2001) rejected this hypothesis, possibly because of the less developed technology used for diagnostic assessment. The very recent implementation of techniques such as optical coherence tomography serve to accurately, instantly and non invasively monitor the progression of the retinal neurodegenerative process, and this fact is important for knowing the decisive effect of natural pigments that absorb harmful radiations.

Several techniques have been also developed to protect eyes subjected to cataract surgery from short wavelength emissions:
- There are several types of filters on the market containing a yellow dye, however no optimal process and/or device has been obtained for applying these filters to the human eye as a preventive and therapeutic measure to substitute and/or to improve the eye's natural protection.
- Since the mid 1990s, intraocular lenses with a yellow filter have been used in eyes subjected to cataract surgery. This alternative involves surgical intervention with all its associated risks and difficulties. Moreover, there is a large group of people subjected to cataract surgery with an implanted transparent intraocular lens substituting the crystalline lens, which lacks the yellow pigment necessary for protection. In these cases, the artificial crystalline lens lacking yellow pigment must be complemented with the intervention of a support system for the yellow pigment, for example the contact lens object of this patent application.

Several patents related to the state of the art have been filed, but they differ significantly from the present invention:
- Corrective contact lens with therapeutic effect (patent FR2761785), designed to correct myopia and strabismus using dyes in certain areas to stimulate or de-stimulate certain retinal areas.
- Laser safety contact lenses (patent number US20050024583) which, by means of certain coatings or treatments, absorb or reflect certain wavelengths and contain one or more identification areas so as to assure that the selected lens is the appropriate one for the required use.
- Colour contact lens for cataracts (patent number JP11253480), designed to solve sunglass problems, consisting of a pupil, like those of colour lenses imitating the effects of a solar glass and a coloured iris with the iris colour patterns.
- Coloured contact lens (patent number JP3294819), designed to protect the eye from light and easily found in case of accidental dropping. It is formed by a glass body or other coloured elements with a translucent colour.
- Contact lens to correct cyanopsia or blue vision illness (patent number JP1204668) by means of introducing a composition of yellow or orange colour capable of absorbing wavelengths between 320-450 nm, for the purpose of reducing the effects of this pathology. Cyanopsia is a perception disorder giving rise to distorted colour vision. Patients see everything tinted with blue; the lens seeks to decrease this perception effect, and by no means does it intend to preserve the retinal neurons from the harmful actions of short wavelength radiation of less than 550 nm.
- Contact lens coloured in certain areas (patent number EP0204347), for patients with retinitis pigmentosa, covering the cornea with an evenly coloured surface such that the maximum transmission of wavelengths of 520 nm is 5% and increases to a minimum of 50% up to values of 660 nm.

These patents differ fundamentally from the present invention in their purpose and utility, since none is designed to protect eyes subjected to cataract surgery and/or a neurodegenerative process against short wavelength radiation.

### Description of the Invention

The objective of the invention in the case of pseudo-aphakic subjects is to functionally compensate the extraction of protective pigments (extracted during surgery), and in the case of neurodegenerative processes, to enhance the prophylactic effect of blue and violet light absorption using a contact lens as a support. As mentioned above, it is very common for these two conditions to coexist in the same population group, i.e. the elderly.

To that end, the invention consists of a therapeutic contact lens for the treatment of eyes undergoing neurodegenerative processes and/or pseudo-aphakic eyes obtained as a result of applying a filter with yellow pigment which absorbs short wavelength radiation of 350-500 nm to a contact lens.

The therapeutic contact lens for pseudo-aphakic eyes therefore consists of the combination of two elements:
- A standard contact lens compatible with the use in the human eye formed by the body of the lens, the iris area and the pupil area.
- The application of a filter with a yellow pigment available on the market which is compatible with the lens and innocuous for the human eye, absorbing short wavelengths from 350 to 500 nm, across the whole area of the lens - including body, iris area and pupil area, resulting in a yellow coloured contact lens. The lens can be provided with different radius curvatures, diameters and refractive powers suitable for each individual.

### Embodiment of the Invention

The possible embodiments of a coloured contact lens are very well known by specialists in the field and the most common methods are the objects of patents US4582402 and US5414477. The embodiment of the present invention is additionally illustrated in the following example which, however, does not limit the scope thereof as there are alternative forms and combinations for the manufacture of the lens:
Example for the production of the invention:
   - 10.3 mg of a conventional yellow dye, 4-phenylazophenol, or Solvent Yellow 7 (SY7), are dissolved in 10.01 g of a monomer solution containing 66% PEA, 30.5% PEMA and 3.3% BDDA, resulting in an SY7 concentration of 0.103 wt%.
   - 52.3 mg of bi 4-tert-butylcyclohexyl peroxydicarbonate is then added as the polymerisation catalyst.
   - Using a syringe, the solution is introduced in a mould formed by two overlapping glass plates joined by metal clips, and a 1 mm Teflon ring. The solution is extended in 1 mm films.
   - Polymerisation takes place when introducing the mould in an oven at 65°C for 17 hours. The oven temperature is later increased to up to 100°C for 3 additional hours.
   - Once polymerisation is complete, the film is extracted from the mould, the appropriate measurement checks are performed and it is subjected to the final cut.

In summary, the combination of a contact lens and a yellow dye, both suitable for the human eye, will allow patients subjected to cataract surgery with a transparent intraocular lens to correct the deprotection of the operated eye through the simple use therein of a contact lens and will also allow the eye undergoing neurodegenerative processes to improve and thus increase natural protection of the eye. Drawbacks associated with the alternative techniques existing on the market (filters without an application device and intraocular lenses) are thus avoided.

## Claims

1. A therapeutic contact lens for pseudo-aphakic eyes **characterized by** resulting from applying a filter with yellow pigment absorbing short wavelengths of 350-500 nm to a contact lens.

2. A therapeutic contact lens for pseudo-aphakic eyes according to claim 1, comprising a filter with yellow pigment suitable for use in the human eye.

3. A therapeutic contact lens for pseudo-aphakic eyes according to claims 1 and 2, comprising a contact lens having features that are compatible for use in the human eye.

4. A therapeutic contact lens for eyes undergoing retinal neurodegenerative processes, **characterized by** resulting from the combination of applying a filter with yellow pigment absorbing short wavelengths of 350-500 nm to a contact lens.

5. A therapeutic contact lens for eyes undergoing retinal neurodegenerative processes according to claim 4, comprising a filter with yellow pigment suitable for use in the human eye.

6. A therapeutic contact lens for eyes undergoing retinal neurodegenerative processes according to claims 4 and 5, comprising a contact lens having features that are compatible for use in the human eye.
